# EUROPEAN PATENT APPLICATION

(11) **EP 4 011 369 A1**
(43) Date of publication of application: **15.06.2022**
(21) Application number: 20213854.1
(22) Date of filing: 14.12.2020
(51) Int. Cl.: A61K 31/137, A61K 31/194, A61K 9/00, A61K 47/12, A61K 47/26, A61P 25/04, A61K 9/08

(54) **AQUEOUS PHARMACEUTICAL COMPOSITION COMPRISING TAPENTADOL TARTRATE**

(71) Applicant: G.L. Pharma GmbH, 8502 Lannach (AT)
(72) Inventor: RAMER, Wolfgang, 8502 Lannach (AT); KROTSCHECK, Robert, 8502 Lannach (AT); WEISI, Daniela, 8502 Lannach (AT)
(74) Representative: SONN Patentanwälte OG

(57) **Abstract**

The present invention relates to an aqueous pharmaceutical composition with a pH value within the range of 4.0 to 5.5, wherein the composition comprises tapentadol tartrate, wherein the composition does not comprise a preservative, and wherein the composition does not comprise a buffer.

## Description

The present invention relates to an aqueous pharmaceutical composition comprising tapentadol tartrate which exhibits excellent stability and efficacy of antimicrobial preservation in the absence of a preservative and in the absence of a buffer.

Tapentadol is a centrally acting analgesic that combines two mechanisms of actions, namely µ-opioid receptor (MOR) agonism and noradrenaline reuptake inhibition (NRI). Pharmacological antagonism studies have demonstrated that both mechanisms of action contribute to the analgesic effects of tapentadol.

It is of primary importance to stabilize the active pharmaceutical ingredient (API) in the final product. As the stability of drug substances in aqueous media is generally lower than the stability of solid dosage forms, the stabilization and preservation of liquid aqueous formulations such as solutions, suspensions, and emulsions is crucial. In such formulations, chemical reactions such as acid or base catalysis, oxidation, reduction and acid-base reactions are likely to occur. These reactions can be related to interactions between the drug substance, excipients, other ingredients and the pack or delivery device, and may change the pH value especially for pH sensitive compounds, which may cause precipitation.

Auto-oxidative reactions can be initiated by heat, light, peroxides, or other labile compounds or heavy metals such as copper or iron. Especially oxidative labile drug substances, vitamins, and most oils and fats are prone to auto-oxidation which leads to chemical degradation. Oxidation can be retarded by employing antioxidants which rapidly react with free radicals. Common antioxidants include butylated hydroxytoluene (BHT), propionic acid, propyl gallate, butylated hydroxyanisole (BHA), propyl, octyl and dodecylesters of gallic acid, sodium ascorbate, sodium sulfite, sodium bisulfite, potassium or sodium metabisulfite, ascorbic acid, monothioglycerol, and the tocopherols or vitamin E.

Apart from stabilization against chemical and physical degradation, pharmaceutical formulations, in particular liquid and semisolid formulations, need to be protected against microbial contamination during shelf-life, particularly when a dose is taken from a multiple dosed formulation. In presence of a sufficient amount of water in the formulation, growth of microorganisms is likely to occur. Thus, contrary to solid dosage forms, aqueous solutions, syrups, emulsions, and suspensions represent an excellent growth media for microorganisms such as bacteria, spore forms, mold and fungi (e.g. Staphylococcus aureus, Escherichia coli, Pseudomonas aeruginosa, Candida albicans, Zygosaccharomyces rouxii and Aspergillus brasiliensis).

Pharmaceutical formulations are typically sterilized to effectively eradicate these microorganisms, mainly by sterilization filtration and aseptic filling or by steam sterilization in the sealed containers. However, especially for multiple dosed formulations, the presence of an antimicrobial preservative may still be required to ensure aseptic conditions throughout the shelf life. The selection of a preservative is based upon several considerations. Not only the antimicrobial activity but also the chemical and physical stability, sensory effects (e.g. irritancy, bitterness) and interactions with the drug, other ingredients in the formulation or with the package or delivery device need to be regarded. Also, when used in liquid and semisolid formulations, the preservative needs to have sufficient aqueous solubility and remain in the aqueous phase throughout the shelf-life of the pharmaceutical product. Furthermore, the pH value of the pharmaceutical product needs to be taken into account, as different preservatives exhibit different ranges for optimum activity.

Thus, the preservative is required to (i) possess a good antimicrobial activity against the microbial species listed in pharmacopeial test methods, (ii) be free from sensory effects, (iii) be compatible with other ingredients of the formulation without showing any adverse interactions, and (iv) maintain its preservative efficacy, chemical and physical stability throughout the shelf-life and in-use period of the pharmaceutical product. To meet all these requirements, a combination of several preservatives is often used.

However, preservatives may cause undesired side effects, such as benzalkonium chloride and potassium sorbate (e.g. used in nasal drops and sprays) which were reported to cause mucosal damage. Quaternary ammoniums, such as benzalkonium chloride, tend to cause irritant toxic reactions, whilst organomercurials, such as thimerosal, and alcohols, such as chlorobutanol, often lead to allergic responses. Further, parabens may lead to contact sensitivity and have been reported to exert a weak estrogenic activity.

Certain applications even require the use of preservative-free pharmaceutical formulations. For example, due to the lack of availability of preservatives that possess sufficient safety, efficacy and non-irritancy to allow its use in pharmaceutical formulations that are used for instillation into ocular or intrathecal tissue, such formulations must not contain any preservative. Furthermore, the assessment of intrinsic safety and labelling is becoming increasingly strict, particularly in Europe, leading to a negative view of some of the more commonly used preservatives (e.g. parabens), with preservative-free medications being promoted as safer products.

Due to these above-mentioned difficulties associated with known preservatives, namely their negative side effects along with the necessity to meet all requirements and safety standards, it is preferred to provide pharmaceutical formulations that provide sufficient stability throughout the shelf-life and in-use period without a preservative.

In the state of the art, tapentadol is commonly used as free base or as a physically acceptable salt thereof in pharmaceutical compositions. The preparation of the free base of tapentadol is known from EP 0 693 475 B1. The documents US 2011/071120 A1 and WO 2017/085734 A1 disclose solid state forms of tapentadol salts and crystalline forms of tapentadol salts, respectively, and a process for their preparation.

EP 3 287 123 B1 relates to an aqueous pharmaceutical composition comprising tapentadol or a physically acceptable salt thereof which is adapted for oral administration. The composition contains a preservative with a content of at most 90% of the content that would be needed according to the European Pharmacopoeia (Ph. Eur.) whilst still sufficiently preserving the pharmaceutical composition.

EP 2 680 832 B1 discloses an aqueous pharmaceutical composition comprising tapentadol free base or the hydrochloride salt thereof which has an excellent storage stability without the addition of a preservative.

It is known that the antimicrobial activity of tapentadol depends on the pH value (EP 3 287 123 B1). To maintain a stable pH value, pharmaceutical compositions are usually buffered. Phosphate buffers are commonly used. DE 19648650 A1 discloses a buffer system comprising a physiologically compatible amine and a physiologically compatible organic acid.

It is an object of the present invention to provide an aqueous pharmaceutical composition comprising tapentadol and a reduced number of other ingredients in order to facilitate the production and avoid undesired side effects, whilst maintaining pH stability and a good stability against bacteria and fungi.

It was now surprisingly found that aqueous pharmaceutical compositions with a pH value within the narrow range of 4.0 to 5.5 comprising tapentadol tartrate are capable of maintaining their pH value even in the absence of a buffer and that such aqueous pharmaceutical compositions exhibit an excellent efficacy of antimicrobial preservation and excellent storage stability in the absence of a preservative.

The present invention is directed to an aqueous pharmaceutical composition with a pH value within the range of 4.0 to 5.5, more preferably 4.0 to 5.0, even more preferably 4.25 to 4.75, wherein the composition comprises tapentadol tartrate, wherein the composition does not comprise a preservative, and wherein the composition does not comprise a buffer. The inventive composition surprisingly meets the criteria of efficacy of antimicrobial preservation as required by the standard Ph. Eur. 5.1.3.

All of the preferred embodiments of this invention are interrelated, and each preferred embodiment and/or disclosed characteristic feature may be combined with each other and also as any combination of two or more preferred embodiments/characteristic features.

The pH value of the pharmaceutical composition according to the present invention (measured at a temperature of 20 °C according to Ph.Eur. 2.2.3) can be adjusted in any suitable manner by means of the addition of pH adjusting agents known in the prior art in an amount sufficient to provide a pH value of the composition at the desired range of 4.0 to 5.5, more preferably 4.0 to 5.0, even more preferably 4.25 to 4.75. For example, the pH value can be adjusted to a lower pH by addition of aqueous hydrochloric acid solutions or to a higher pH by adding aqueous sodium hydroxide solutions. Such solutions can be diluted or concentrated.

The term 'pharmaceutical composition' as used in the context of the present invention is to be understood to comprise any pharmaceutical preparation or formulation that is customized for being administered to a human being or animal. Preferably, the composition contains one or more physiologically acceptable carriers, preferably water and/or excipients. The pharmaceutical composition may also be a subunit of a pharmaceutical dosage form, e.g. the liquid core of a capsule.

The term 'aqueous' as used in the context of the present invention refers to any solution in which water is either the only solvent or one of the main solvents (equal or above 50% v/v). Aqueous solutions include, but are not limited to solutions comprising 50%, 60%, 70%, 80%, 85%, 90%, 95%, 98%, 99% v/v water. The aqueous solutions can additionally comprise pharmaceutically acceptable and physiologically tolerated organic solvents, either one or more, that are normally liquid at ambient or room temperature. Such organic solvents include glycols, such as polyethylene glycol (PEG 200, PEG 300, PEG 400, PEG 600, PEG 4000, etc.) and propylene glycol, alcohols such as ethanol, glycol monoethyl ether, glycofurol, glycerol, propylene carbonate, or any mixtures thereof. Said further solvent may also include ionic tensides, non-ionic tensides, emulsifiers and/or fats. Suitable anionic tensides may be selected from the group comprising sodium cetyl stearyl sulfate, sodium dioctylsulfosuccinate, sodium lauryl sulfate, and the corresponding calcium or potassium salts thereof. Suitable fats may be selected from glycerol monostearate, glycerol monopalmitate, stearic acid, diglycol stearate, glycerol trioleate, carnauba wax, bees wax, cetylstearyl alcohol and the like. The aqueous solution(s) according to the present invention may further comprise diluents selected from a group consisting of sterile water, bacteriostatic water for injection (BWFI), and any mixtures thereof.

Within the present application, the term 'preservative' is to be understood to comprise any substance that is added to pharmaceutical compositions with the purpose to preserve these compositions against microbial degradation or microbial growth. In this regard, microbial growth typically plays an essential role, i.e. the preservative serves the purpose of avoiding microbial contamination of the pharmaceutical composition. As a side aspect, it may also be desirable to avoid any effect of the microbes on the active ingredients and excipients, respectively, i.e. to avoid microbial degradation. Examples of preservatives comprise chloride in general, benzethonium chloride, benzoic acid, sodium benzoate, benzyl alcohol, bronopol, cetrimide, cetylpyridinium chloride, chlorhexidine, chlorbutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, sodium propionate, thimerosal, methyl paraben, ethyl paraben, propyl paraben, butyl paraben, isobutyl paraben, benzyl paraben, sorbic acid, and potassium sorbate.

Within the present application, the term 'shelf life' or 'storage stability' refers to the storage stability of a closed container, package or delivery device of the pharmaceutical composition. The term 'in-use stability' refers to the storage container that comprises a multiple dose preparation which has been utilized for the first time.

The concentration of tapentadol used in the inventive pharmaceutical composition is within the range of 15 to 25 mg/ml, preferably 20 mg/ml calculated as free base. The corresponding concentration of tapentadol tartrate used in the inventive pharmaceutical composition is within the range of 25 to 42 mg/ml, preferably 33.6 mg/ml. Within this range of concentration, the handling of the inventive pharmaceutical composition is facilitated and further preparation steps prior to the administration, such as dilution, are not required, thus eliminating the possibility of polluting the composition shortly before administration.

Within the present application, the term 'buffer' is to be understood to comprise one or more pharmaceutically acceptable and physiologically tolerated buffers and/or buffer systems, i.e. conjugate acid-base-pairs, which are added to pharmaceutical compositions with the purpose to maintain a certain pH value throughout the shelf life, thus ensuring storage stability and chemical stability. Preferred buffer systems are derived from the following acids: organic acids, such as acetic acid, propionic acid, maleic acid, fumaric acid, malonic acid, malic acid, mandelic acid, citric acid, tartaric acid; or inorganic acids, such as phosphoric acid. When the buffer systems are derived from any of the above acids, the buffer system constitutes of said acid and its conjugate base.

In a preferred embodiment, the composition comprises a citrate buffer comprising citric acid and an adequate base, e.g. sodium hydroxide solution or citric acid and a citrate salt. Such a citrate buffer is highly effective in the pH range of the inventive aqueous pharmaceutical composition, well soluble in the composition, shows no significant interactions with the other ingredients, is chemically stable throughout the shelf life and physiologically well tolerated without showing any negative side effects. Preferably, the concentration of the citrate buffer, is within the range of 10 to 20 mmol/l, particularly preferably 15 mmol/l, which is an optimal range of concentration to maintain a stable pH value throughout the shelf life. Preferably, the citric acid is added in form of citric acid monohydrate with the formula C₆H₈O₇ · H₂O. Preferably, the concentration of the citric acid is within the range of 1.6 to 2.6 mg/ml, particularly preferably 2.08 mg/ml, based on the total volume of the composition. Within the scope of the present invention, the term 'citrate salt' is to be understood to comprise all pharmaceutically acceptable and physiologically tolerated salts of citric acid, such as sodium citrate, potassium citrate, magnesium citrate and calcium citrate. In a preferred embodiment, the citrate salt used in the inventive pharmaceutical composition is sodium citrate. Preferably, the concentration of the citrate, preferably the sodium citrate, is within the range of 1.0 to 2.0 mg/ml, more preferably within the range of 1.2 to 1.7 mg/ml, particularly preferably 1.5 mg/ml, based on the total volume of the composition.

The pharmaceutical composition according to the present invention may additionally comprise a taste-enhancing component. In a preferred embodiment, the taste-enhancing component comprises at least one sweetener, preferably selected from the group comprising cyclamate (E 952), saccharin (E 954) or sodium saccharin, aspartame (E 951), sucralose (E 955), neotame, thaumatine (E 957), neohesperidine (E 959), acesulphame potassium (acesulphame K, E 950) and acesulphame-aspartame salt (E 962), and sorbitol (E 420), most preferably sucralose. Preferably, the concentration of the sweetener is within the range of 1.0 to 2.0 mg/ml, particularly preferably 1.5 mg/ml, based on the total volume of the composition.

The composition according to the present invention may additionally comprise a flavor or aromatic additive. Said flavor or aromatic additive preferably comprises at least one natural fruit flavor, such as strawberry flavor, raspberry flavor and currant flavor, particularly preferably raspberry flavor. Preferably, the concentration of the flavor or aromatic additive is within the range of 0.1 to 1.0 mg/ml, particularly preferably 0.6 mg/ml.

The composition according to the present invention may additionally comprise one or more pharmaceutically acceptable and physiologically tolerated excipients selected from the group comprising wetting agents, emulsifying agents, isotonizing agents, surfactant components, solubilizing agents, thickening agents, colorant agents and antioxidant components.

The composition according to the present invention is adapted for oral administration. Within the present application, the term 'oral administration' comprises every administration through the oral cavity, such as buccal, peroral, sublingual, and the like. In order to minimize the effort for the health care professionals and a potential contamination risk, the aqueous pharmaceutical composition according to the present invention is preferably provided as a ready-to-use formulation and does not require any particular steps of treatment, such as dissolution in a solvent, before it may be orally administered. It is also possible to provide the composition as a multiple dosed preparation. In the context of this application, the term 'multiple dosed' means that the composition comprises more than a single dosage unit. For example, when the composition is a multiple dosed oral solution, its overall volume is more than the volume that is to be typically orally administered at once. The inventive composition can further be provided as a pharmaceutical dosage form selected from the group comprising oral solutions, oral gels, suspensions, emulsions, and liquid or gel filled capsules.

In a preferred embodiment, the composition is used for the treatment of pain, such as acute pain, chronic pain, inflammatory pain, neuropathic pain, visceral pain, cancer pain and migraine pain.

### Examples

The following examples are supposed to further illustrate the invention as described within this application without any intention to limit the scope of the invention.

The recipes of four pharmaceutical compositions according to the present invention are listed in Table 1. The preparation of the compositions was performed at a temperature in the range of 18 °C to 25 °C. Tapentadol tartrate was dissolved in approximately 90% of the total amount of purified water (i.e., deionized, demineralized and sterilized water) upon stirring. 2M sodium hydroxide solution was added to the target pH, Sucralose Ph.Eur. and Raspberry Symrise SY 203580 (Symrise, Holzminden, Germany) were added upon stirring. When appropriate, citric acid monohydrate and trisodium citrate were also added. Subsequently, purified water was added up to the final weight of 1000 ml. The compositions were passed through a pharmaceutical-grade filter membrane, divided into glass bottles and sealed with an adequate, child-resistant closure.

**Table 1: Recipes of pharmaceutical compositions according to the present invention (contents in g) and their pH value**

| | **without buffer** | | **with buffer** | |
|---|---|---|---|---|
| **Composition no.** | **1** | **2** | **3** | **4** |
| Aqua pur. I, cold | 900 | 900 | 900 | 900 |
| **Trisodium citrate** | 0 | 0 | 1.5 | 1.5 |
| Citric acid monohydrate | 0 | 0 | 2.08 | 2.08 |
| Sucralose | 1.5 | 1.5 | 1.5 | 1.5 |
| Tapentadol tartrate | 33.56 | 33.56 | 33.56 | 33.56 |
| Raspberry Symrise SY 203580 | 0.6 | 0.6 | 0.6 | 0.6 |
| **2M NaOH ad pH** | **4.0** | **5.5** | **4.0** | **5.5** |
| Aqua pur. II, ad 1000 ml | | | | |
| **TOTAL content** | **1000** | **1000** | **1000** | **1000** |

The efficacy of antimicrobial preservation was assessed according to Ph. Eur. 5.1.3 - 3 - Oralia. Six test strains were analyzed, namely staphylococcus aureus (ATCC 6538), pseudomonas aeruginosa (ATCC 9027), candida albicans (ATCC 10231), aspergillus brasiliensis (ATCC 16404), escherichia coli (ATCC 8739) and zygosaccharomyces rouxii (NCYC 381). A series of containers of the compositions to be examined was inoculated, each with one of the reference strains to result to 10⁵ to 10⁶ microorganisms per ml or g of preparation. The volume of inoculum was less or equal to 1% of the volume of the product. The inoculated product was incubated at 20 °C to 25° C (fungi) and at 30 °C to 35 °C (bacteria).

Samples from each of the inoculated containers were taken at 0 h (referred to as 'immediate'), 7 days and 14 days. The number of viable microorganisms was determined according to Ph. Eur. 2.6.12 (Microbiological examination of non-sterile products) using the pour plate method (1 ml and 0.1 ml). If the microbial count was < 100 CFU/ml (inoculated product), the membrane filtration method was performed for the subsequent time points. The efficacy of antimicrobial preservation (according to Ph.Eur. 5.1.3) of the analyzed compositions and a control sample (water) is listed in Table 2.

It can be clearly seen from Table 2 that all analyzed pharmaceutical compositions according to the invention meet the criteria of efficacy of antimicrobial preservation as required by the standard Ph. Eur. 5.1.3. In terms of the efficacy of antibacterial preservation, the microbial count after 7 days is below < 100 CFU/ml or non-detectable (listed as '0' in Table 2), whilst it is non-detectable after 14 days for all compositions at both investigated pH values of 4.0 and 5.5.

Contrary, the efficacy of antifungal preservation is less pronounced for all compositions. Surprisingly, an increase of the pH value from 4.0 to 5.5 significantly enhances the efficacy of antimicrobial preservation, with the microbial count being below < 100 CFU/ml after 7 days and non-detectable after 14 days.

These results clearly show that the efficacy of antimicrobial preservation is significantly affected by the pH value. At a pH value of 5.5, an excellent efficacy of antimicrobial preservation, i.e. antibacterial and antifungal preservation, is achieved with pharmaceutical compositions according to the present invention even in absence of a preservative and in absence of a buffer.

**Table 2: Efficacy of antimicrobial preservation (according to Ph.Eur. 5.1.3 - 3 - Oralia) of pharmaceutical compositions according to the present invention and a control (numbers in CFU/ml)**

| | **Bacteria** | | | **Fungi** | | |
|---|---|---|---|---|---|---|
| | **Staphylococcus aureus** | **Pseudomonas aeruginosa** | **Escherichia coli** | **Candida albicans** | **Zygosaccharomyces rouxii** | **Aspergillus brasiliensis** |
| **Control** | | | | | | |
| immediate | 1.5 · 10⁶ | 2.0 · 10⁶ | 1.8 · 10⁶ | 1.5 · 10⁶ | 1.7 · 10⁶ | 1.1 . 10⁵ |
| after 28 days | 1.4 · 10⁶ | 2.0 · 10⁶ | 1.6 · 10⁶ | 1.3 · 10⁶ | 1.7 · 10⁶ | 1.0 · 10⁵ |

| **Sample 1 (pH = 4.0, without buffer)** | | | | | | |
|---|---|---|---|---|---|---|
| immediate | 1.3 · 10⁶ | < 100 | 1.5 · 10⁶ | 1.2 · 10⁶ | 1.6 · 10⁶ | 1.1 . 10⁵ |
| after 7 days | < 100 | 0 | < 100 | 1.0 · 10² | 1.5 · 10² | 1.0 · 10³ |
| after 14 days | 0 | 0 | 0 | 10 | 15 | 1.0 · 10³ |

| **Sample 2 (pH = 5.5, without buffer)** | | | | | | |
|---|---|---|---|---|---|---|
| immediate | 1.5 · 10⁶ | < 100 | 1.5 · 10⁶ | 1.3 · 10⁶ | 1.7 · 10⁶ | 1.0 · 10⁵ |
| after 7 days | < 100 | 0 | < 100 | < 100 | < 100 | < 100 |
| after 14 days | 0 | 0 | 0 | 0 | 0 | 0 |

| **Sample 3 (pH = 4.0, with buffer)** | | | | | | |
|---|---|---|---|---|---|---|
| immediate | 1.5 · 10⁶ | < 100 | 2.0 · 10⁶ | 1.5 · 10⁶ | 1.5 · 10⁶ | 1.2 · 10⁵ |
| after 7 days | < 100 | 0 | < 100 | 2.5 · 10² | 3.0 · 10² | 4.0 · 10⁴ |
| after 14 days | 0 | 0 | 0 | 25 | 25 | 2.0 · 10³ |

| **Sample 4 (pH = 5.5, with buffer)** | | | | | | |
|---|---|---|---|---|---|---|
| immediate | 1.8 · 10⁶ | < 100 | 1.5 · 10⁶ | 1.0 . 10⁶ | 1.6 . 10⁶ | 1.0 · 10⁵ |
| after 7 days | < 100 | 0 | < 100 | < 100 | < 100 | < 100 |
| after 14 days | 0 | 0 | 0 | 0 | 0 | 0 |

The stability of the pharmaceutical compositions after 6 weeks of storage at different temperatures (25 °C, 40 °C and 60 °C), namely the HPLC assay and impurities (also referred to as degradation products), were determined by means of HPLC analysis according to Ph.Eur. 10.2 monograph 3035 ("tapentadol hydrochloride") and is listed in Table 3. The compositions exhibit an excellent chemical stability at all analyzed temperatures in the presence or absence of a buffer and for both investigated pH values of 4.0 and 5.5.

**Table 3: Stability of the pharmaceutical compositions after 6 weeks of storage at different temperatures as determined by HPLC analysis according to Ph.Eur. 10.2 monograph 3035 ("tapentadol hydrochloride")**

| | **Temperature (°C)** | **pH value (-)** | **Assay (%)** | **Degradation products (%)** |
|---|---|---|---|---|
| **Sample 1** (pH = 4.0, without buffer) | 25 | 4.00 | 99.8 | < 0.01 |
| | 40 | 3.99 | 100.2 | < 0.01 |
| | 60 | 3.99 | 99.8 | < 0.01 |
| **Sample 2** (pH = 5.5, without buffer) | 25 | 5.54 | 99.1 | < 0.01 |
| | 40 | 5.54 | 99.2 | < 0.01 |
| | 60 | 5.54 | 98.8 | < 0.01 |
| **Sample 3** (pH = 4.0, with buffer) | 25 | 4.01 | 100.2 | < 0.01 |
| | 40 | 4.00 | 98.3 | < 0.01 |
| | 60 | 4.00 | 99.6 | < 0.01 |
| **Sample 4** (pH = 5.5, with buffer) | 25 | 5.52 | 100.3 | < 0.01 |
| | 40 | 5.53 | 100.2 | < 0.01 |
| | 60 | 5.53 | 99.6 | < 0.01 |

## Claims

1. Aqueous pharmaceutical composition with a pH value within the range of 4.0 to 5.5, wherein the composition comprises tapentadol tartrate, wherein the composition does not comprise a preservative and wherein the composition does not comprise a buffer.

2. The composition according to claim 1, wherein the concentration of tapentadol is 20 mg/ml calculated as free base, based on the total volume of the composition.

3. The composition according to claim 1 or 2, wherein the composition additionally comprises a citrate buffer.

4. The composition according to claim 3, wherein the concentration of the citrate buffer is within the range of 10 to 20 mmol/l, based on the total volume of the composition.
